# EUROPÄISCHE PATENTSCHRIFT

(11) **EP 0 889 048 B1**
(45) Veröffentlichungstag und Bekanntmachung des Hinweises auf die Patenterteilung: **03.04.2002**
(21) Anmeldenummer: 98109834.6
(22) Anmeldetag: 29.05.1998
(51) Int. Cl.: C07F 9/6568, C07F 15/00, B01J 31/24, C07B 53/00, C07C 45/50

(54) **Herstellung optisch aktiver Phospholane, deren Metallkomplexe und Anwendung in der asymmetrischen Synthese**
Preparation of optically active phospholanes, their metal complexes and their use in asymmetric synthesis
Préparation de phospholanes optiquement actifs, leurs complexes avec des métaux et leur utilisation dans la synthèse asymétrique

(30) Priorität: 18.06.1997 DE 19725796
(43) Veröffentlichungstag der Anmeldung: 07.01.1999
(73) Patentinhaber: BASF AKTIENGESELLSCHAFT, 67056 Ludwigshafen (DE); Max-Planck-Gesellschaft zur Förderung der Wissenschaften e.V., 80539 München (DE)
(72) Erfinder: Stürmer, Rainer, Dr., 67127 Rödersheim-Gronau (DE); Börner, Armin, Dr., 18059 Rostock (DE); Holz, Jens, Dr., 18057 Rostock (DE); Voss, Gudrun, 18055 Rostock (DE)

(56) Entgegenhaltungen:
- WO-A-91/17998
- WO-A-92/19630
- H. BRUNNER: "Asymmetric Calalyses XXXIII. New optically active phospholanes derived from tartaric acid" JOURNAL OF ORGANOMETALLIC CHEMISTRY., Bd. 328, Nr. 1-2, - 14. Juli 1987 Seiten 71-80, XP002077949 LAUSANNE CH

## Beschreibung

Die Erfindung beschreibt neue optisch aktive Phospholane und Bis-Phospholane, deren Herstellung und Verwendung als Liganden in Metallkomplexen sowie die Anwendung der Metallkomplexe für die enantioselektive Synthese.

Für die Synthese optisch aktiver Verbindungen spielt die enantioselektive Hydrierung und Isomerisierung mit Rhodium und Rutheniumkomplexen eine grosse Rolle (z.B. Tani et al. J. Am. Chem Soc 106, 5211, 1984; R. Noyori, Acc. Chem. Res. 23, 345 (1990)) Der stöchiometrische Einsatzstoff Wasserstoff ist billig, allerdings sind die eingesetzten Katalysatoren, die meist aus einem optisch aktiven Diphosphinliganden und einer Rhodium oder Rutheniumverbindung hergestellt werden, sehr teuer und nur aufwendig zugänglich.

Die bekannten Herstellmethoden für optisch aktive Phosphine und Diphosphine sind durchweg komplex und schliessen meist eine technisch aufwendige und teure Racematspaltung ein (z.B. EP-A 0614901; EP-A 0271311; H.B. Kagan, "Chiral Ligands for Asymmetric Catalysis" in Asymmetric Synthesis, Vol. 5 (1985), Seite 13 - 23, EP-A 0151282; EP-A 0185882; R. Noyori, Acc. Chem. Res. 23, 345 (1990); EP- 269395; M.J. Burk, Tetrahedron, Asymmetry, S. 569 - 592 (1991); J. Am. Chem. Soc. 113, S. 8518 - 9 (1991), ibid. 115, S. 10125 - 138 (1993), ibid. 117, S. 9375 - 76 (1995), ibid 118, S. 5142 (1996). Diese Nachteile machen eine industrielle Nutzung schwierig und unwirtschaftlich.

Aufgabe der vorliegenden Erfindung war es daher, Phosphinliganden bereitzustellen, die sich leicht und kostengünstig herstellen lassen und die gute Liganden für Metallkomplexkatalysatoren zur enantioselektiven Synthese darstellen.

Es wurde nun gefunden, dass eine besonders effiziente Klasse von Liganden, namentlich Phospholane aus dem "Chiral Pool" zugänglich ist. Das Ausgangsmaterial ist in diesem Fall Mannit und andere Kohlenhydrate, die in grosser Menge billig verfügbar sind.

Die erhaltenen Phospholane und Diphospholane liefern in asymmetrischen Hydrierungen ausgezeichnete Enantiomerenüberschüsse. Bekannt sind die DUPHOS - Liganden von Burk et. al. (M.J. Burk, Tetrahedron, Asymmetry, S. 569 - 592 (1991); J. Am. Chem. Soc. 113, S. 8518 - 9 (1991), ibid. 115, S.10125 - 138 (1993), ibid. 117, S. 9375 - 76 (1995), ibid 118, S. 5142 (1996)) die im Gegensatz zur vorliegenden Erfindung sehr viel aufwendiger zu synthetisieren sind. Zur Synthese der DUPHOS-Liganden sind u.a. eine unpraktische elektrolytische Kolbe-Synthese nebst einer asymmetrischen Hydrierung erforderlich.

Die vorliegende Erfindung umgeht diese Schwierigkeiten durch den Einsatz des Zuckers Mannit, der aus natürlichen Quellen enantiomerenrein verfügbar ist. Ferner eröffnet dieses Edukt einen Weg zu Strukturanalogen, die im Phospholan-Ring in 3 u. 4-Stellung substituiert sind, d.h. zu Analogen , die mit der bekannten DUPHOS-Synthese nicht herzustellen sind.

Gegenstand der Erfindung sind Phospholane und Diphospholane der allgemeinen Formel I 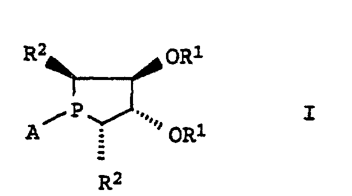 wobei:
- R¹ und R²: unabhängig voneinander C₁-C₆ -Alkyl, Aryl, Alkylaryl,
- R¹: außerdem Wasserstoff,
- A: entweder R¹ oder 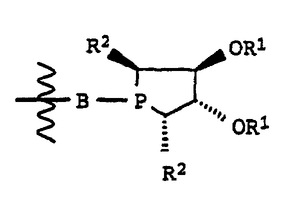
- B: ein Brückenglied mit 1 - 5 C-Atomen zwischen den beiden P-Atomen bedeuten.

Bevorzugte Substituenten R¹ und R² sind Methyl, Ethyl, n-Propyl, Isopropyl, n-Butyl, Isobutyl, tert.Butyl, Benzyl.

Weiterhin auch solche Reste R¹, bei denen die beiden R¹ zusammen Isopropyliden oder Benzyliden bedeuten.

Im Falle der Diphospholane sind solche bevorzugt bei denen 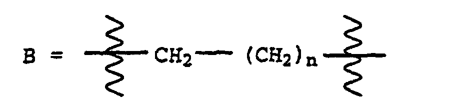 mit n = 0, 1, 2, 3, 4 oder 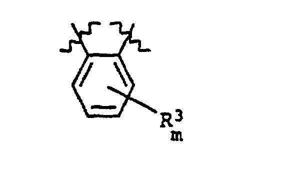 mit
m = 0, 1, 2, 3
R³ = Alkyl oder annelliertes Aryl

Besonders bevorzugt sind solche Brückenglieder B bei denen n=1 oder 2 oder m=0 ist.

Ein weiterer Gegenstand der Erfindung sind Metallkomplexe aus den oben genannten Phospholanen.

Besonders bevorzugte Metallkomplexe sind solche, die als Zentralatom Ruthenium oder Rhodium enthalten. Diese Komplexe lassen sich herstellen, indem man in bekannter Weise (z.B. Uson, Inorg. Chim. Acta 73, 275 (1983), ,EP-A 0158875, EP-A 437690) durch Umsetzung mit Rhodium-, Iridium-, Ruthenium-,Palladium-, Platin-; Nickelkomplexen, die labile Liganden enthalten (z.B. [RuCl₂(COD)]ₙ, Rh(COD)₂BF₄, Rh(COD)₂ClO₄, [Ir(COD)Cl]₂, p-Cymol-Rutheniumchlorid-dimer) katalytisch aktive Komplexe synthetisiert werden.

Ein weiterer Gegenstand der Erfindung ist die Verwendung dieser Metallkomplexe in der asymmetrischen Synthese, insbesondere als Katalysator für Hydrierungen, Hydroformylierungen, Hydrocyanierungen, allylische Substitutionen und Isomerisierungen von Allylaminen zu Enaminen

Diese Reaktionen können mit den erfindungsgemäßen Metallkomplexen unter dem Fachmann geläufigen Bedingungen durchgeführt werden.

Die Hydrierung mit den erfindungsgemäßen Metallkomplexen wird in der Regel bei einer Temperatur von -20 bis 150°C, bevorzugt bei 0 bis 100°C und besonders bevorzugt bei 15 - 40°C durchgeführt.

Der Wasserstoffdruck kann in einem großen Bereich zwischen 0,1 bar und 300 bar für das erfindungsgemäße Hydrierverfahren variiert werden. Sehr gute Ergebnisse erhält man in einem Druckbereich von 1 - 10, bevorzugt 1 - 2 bar.

Besonders vorteilhaft bei den erfindungsgemäßen Liganden ist der niedrige Wasserstoffdruck von 1 - 2 bar, bei dem die Hydrierungen sehr effizient durchgeführt werden können.

### Beispiel 1:

### Darstellung von Tetrabenzyloxy-Me-DUPHOS

1,2;5,6-Di-*O*-isopropyliden-D-mannitol (1): kommerziell erhältlich bei Firma FLUKA (Best.-Nr. 38410).

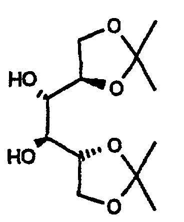

3,4-Di-*O*-benzyl-1,2;5,6-di-O-isopropyliden-D-mannitol (2): dargestellt nach: J. Jurcak, T. Bauer, M. Chmielewski, *Carbohydr. Res.* 164 (1987) 493.

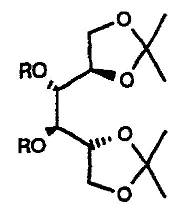

3,4-Di-*O*-benzyl-D-mannitol (3): dargestellt nach: J. Jurcak, T. Bauer, M. Chmielewski, *Carbohydr. Res.* 164 (1987) 493.

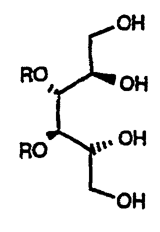

3,4-Di-*O*-benzyl-1,6-di-*O*-toluolsulfonyl-D-mannitol (4): dargestellt nach: J. Fittremann, A. Duréault, J.-C. Depezay, *Tetrahedron Leiters* 35 (1994) 1201.

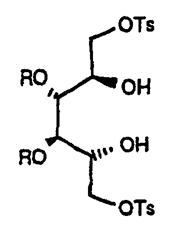

(*2R,3R,4R,5R*)-3,4-Dibenzyloxy-hexan-2,5-diol (5): Eine Lösung bestehend aus 10 g (14.9 mmol) des Ditosylates 4 in 30 ml THF wird bei Raumtemperatur langsam zu einer Suspension von 2.25 g (59.6 mmol) LiAlH₄ in 100 mL THF getropft. Nach einer Stunde Rühren erhitzt man die Suspension zwei Stunden unter Rückfluß. Nach dem Abkühlen zersetzt man das Hydrid durch vorsichtige aufeinanderfolgende Zugabe von 2.25 ml Wasser, 2.25 ml 15 %-iger NaOH und nochmals 6.75 ml Wasser. Die Lösung wird von den ausgefallenen anorganischen Verbindungen abfiltriert und dieser Rückstand mittels Methylenchlorid im Soxhlet extrahiert. Die vereinigten Lösungen werden getrocknet und nach dem Abdestillieren der Lösungsmittel wird der Rückstand säulenchromatographisch gereinigt. (*n*-Hexan:AcOEt = 1:2; R_{f} = 0.45).

Ausbeute: 3.6 g (73 %), weißer Feststoff. Fp.= 46-50°C. [α]²⁶_{D} = -4.7 (c 0.990, CHCl₃), ¹H-NMR (CDCl₃) : 7.40-7.25 (10 H, m, arom. H), 4.65 (4H, AB-Sp., CH₂Ph, ²J_{A,B}= 11.3 Hz), 4.09 (2H, m, H-2+H-5), 3.53 (2H, m, H-3+H-4), 2.96 (2H, s (Br), 2xOH), 1.25 (6H, d, 2×CH₃, ³J_{H,H}= 6.4Hz); ¹³C-NMR (CDCl₃) : 137.4, 128.5, 128.2, 128.0 (arom. C), 81.5 (C-3+C-4), 73.3 (2xCH₂Ph), 67.3 (C-2+C-5), 19.7 (2xCH₃); IR (KBr): 3417, 3287, 3031, 2987, 2965, 2934, 2882, 1455, 1316, 1210, 1112, 1092, 1075, 1056, 1028, 764, 726, 697; MS (70 eV, m/z): 331 [M⁺+H] (1), 297 [M⁺-CH₃-H₂O] (1), 285 [M-C₂H₅O] (2); C₂₀H₂₆O₄ (330.43) ber: C: 72.70 % H: 7.93 %; gef.: C: 72.79 % H: 7.94 %;

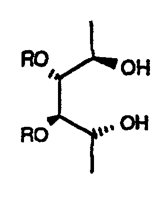

(*4R,5R,6R,7R*)-5,6-Dibenzyloxy-4,7-dimethyl-[1,3,2]dioxathiepan 2,2-dioxid (6): 4.75 g (14.4 mmol) des Diols 5 werden in 20 ml Tetrachlorkohlenstoff mit 1.3 ml Thionylchlorid 1,5 h unter Rückfluß erhitzt. Nach dem Abkühlen entfernt man das Lösungsmittel am Rotationsverdampfer und nimmt den erhaltenen Rückstand mit 10 ml Tetrachlorkohlenstoff, 10 ml Acetonitril und 15 ml Wasser auf. Zu der auf 0°C abgekühlten Lösung gibt man 0.021 g (0.08 mmol) RuCl₃*3H₂O und anschließend 6.2 g (29.0 mmol) Natriumperiodat. Nach einstündigem Rühren bei Raumtemperatur versetzt man die Lösung mit 75 ml Wasser und extrahiert mit 4 x 100 ml Diethylether. Die vereinigten Extrakte werden einmal mit gesättigter NaCl-Lösung gewaschen, anschließend mit Na₂SO₄ getrocknet und durch Kieselgur filtriert. Die etherische Lösung engt man ein und reinigt das cyclische Sulfat 6 durch Säulenchromatographie (n-Hexan : AcOEt = 9 : 1, R_{f} = 0.25).

Ausbeute 3.4 g (60 %) weiße Kristalle. Fp.= 90-94°C. [α]²³_{D} = -2.8 (*c* 1.012, CHCl₃). ¹H-NMR (CDCl₃): 7.40-7.25 (10 H, m, arom. H), 4.79 (4H, AB-Sp., CH₂Ph, ²J_{A,B}= 10.8 Hz), 4.09 (2H, m, H-2+H-5), 3.55 (2H, m, H-3+H-4), 1.53 (6H, d, 2xCH₃, ³J_{H,H}= 6.4Hz); ¹³C-NMR (CDCl₃): 137.1, 128.6, 128.1, 127.7 (arom. C), 84.2 (C-3+C-4), 79.4 (C-2+C-5), 76.2 (2xCH₂Ph), 17.9 (2xCH₃); IR (KBr): 3090, 3062, 3027, 2989, 2939, 2881, 2861, 1498, 1453, 1395, 1380, 1349, 1208, 1103, 1071, 1020, 949, 899, 841, 750, 741, 703, 699, 611; MS (70 eV, m/z): 392 [M⁺] (1), 301 [M⁺-C₇H₇] (47), 195 [M⁺-C₇H₇-C₇H₆O] (36), 91 [C₇H₇⁺] (100); C₂₀H₂₄O₆S (392.47) ber: C: 61.21 % H: 6.16 % S: 8.17 %; gef.: C: 61.20 % H: 6.24 % S: 8.08 %;

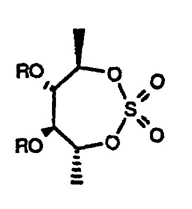

1,2-Bis((*2S,3S,4S,5S*)-3,4-dibenzyloxy-2,5-dimethyl-phospholanyl)benzen (7): Zu einer Lösung von 0.564 g (3.96 mmol) 1,2-Bis(phosphanyl)benzen in 70 ml THF gibt man tropfenweise 4.95 ml (7.93 mmol) *n*-BuLi (1.6 M in Hexan) bei Raumtemperatur. Die entstehende gelbe, klare Lösung wird weitere 2 Stunden gerührt und anschließend langsam mit einer Lösung von 3.11 g (7.92 mmol) des cyclischen Sulfats 6 in 15 ml THF versetzt. Dabei erfolgt ein Farbumschlag nach rot-orange. Nach vier Stunden überführt man weitere 5.45 ml (8.71 mmol) n-BuLi in das Reaktionsgemisch und läßt weitere 16 h bei Raumtemperatur rühren. Zur Aufarbeitung wird die resultierende rote Lösung mit 3 ml Methanol versetzt und das THF unter Vakuum entfernt. Der Rückstand wird mit 50 ml Methylenchlorid aufgenommen und unter anaeroben Bedingungen mit Wasser (20 ml) gewaschen. Nach dem Trocknen (Na₂SO₄) und Entfernen des Lösungsmittels erfolgt eine chromatographische Reinigung (*n*-Hexan : AcOEt = 9 : 1, R_{f}=0.2).

42 % Ausbeute farbloser Sirup. ¹H-NMR (C₆D₆): 7.70-7.00 (10 H, m, arom. H), 4.50 (8H, m, 4xCH₂Ph), 4.05-3.93 (4H, m, H-2+H-5), 3.15-2.94 (4H, m, H-2+H-5), 1.47 (6H, m, CH₃), 0.88 (6H, m, CH₃); ¹³C-NMR (C₆D₆): 143.3 (m), 139.3, 139.3, 128.5-127.5 (arom. C), 85.2+84.2 (C-3+C-4), 72.2+72.0 (4xCH₂Ph), 32.4 (m, C-2+C-5), 14.5 (CH₃), 13.4 (CH₃); ³¹P-NMR (C₆D₆) : -3.4; MS (FDₚₒₛ): 731 [M⁺+H] (100);

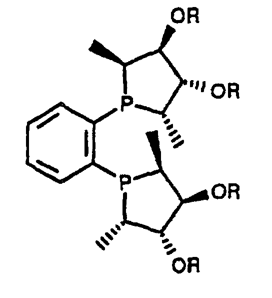

(COD)Rh(1,2-Bis((*2S,3S,4S,5S*)-3,4-dibenzyloxy-2, 5-dimethylphospholanyl)benzen (8): Zu einer Lösung von 300 mg (0.41 mmol) des Phospholans 7 in 3 ml THF gibt man ein Äquivalent (167 mg) [Rh(COD)₂]BF₄ und rührt eine Stunde bei Raumtemperatur. Anschließend versetzt man die Lösung mit 20 ml Diethylether wobei sich ein dunkelbraunes Öl abscheidet. Nach Abtrennen der überstehenden Lösung wäscht man den Rückstand mit Diethylether (3 x 10 ml) und trocknet anschließend unter Vakuum. Dabei erhält man den gewünschten Komplex als braunes Pulver.

Ausbeute 200 mg (47 %). ¹H-NMR (CHCl₃): 7.70-7.00 (10 H, m, arom. H), 4.50 (8H, m, 4xCH₂Ph), 4.05-3.93 (4H, m, H-2+H-5), 3.15-2.94 (4H, m, H-2+H-5), 1.47 (6H, m, CH₃), 0.88 (6H, m, CH₃); ¹³C-NMR (C₆D₆): 143.3 (m), 139.3, 139.3, 128.5-127.5 (arom. C), 85.2+84.2 (C-3+C-4), 72.2+72.0 (4xCH₂Ph), 32.4 (m, C-2+C-5), 14.5 (CH₃), 13.4 (CH₃); ³¹P-NMR (CDCl₃): 76.1 (d, ¹J_{Rh,P}= 152.6 Hz); MS (FDₚₒₛ): 941 [M⁺-BF₄] (100);

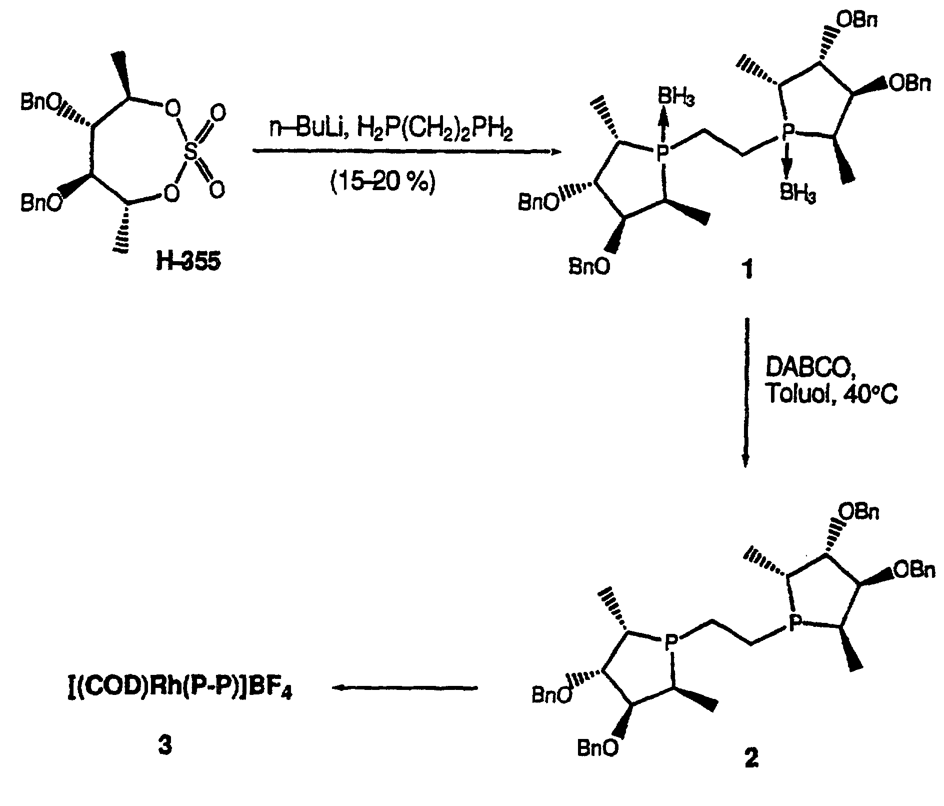

1,2-Bis((*2S,3S,4S,5S*)-3,4-dibenzyloxy-2,5-dimethyl-phospholanyl)benzen (Boran-Komplex) (1): Zu einer Lösung von 0.396 g (4.21 mmol) 1,2-Bis(phosphanyl)ethan in 70 ml THF gibt man bei Raumtemperatur unter Rühren tropfenweise 5.26 ml (8.42 mmol) *n*-BuLi (1.6 M in Hexan). Die entstehende gelbe, klare Lösung wird weitere 2 Stunden gerührt und anschließend gibt man langsam eine Lösung von 3.30 g (7.92 mmol) des cyclischen Sulfats ((*4R,5R,6R,7R*)-5,6-Dibenzyloxy-4,7-dimethyl-[1,3,2]dioxathiepan-2,2-dioxid) gelöst in 15 ml THF hinzu. Dabei erfolgt ein Farbumschlag nach gelb-braun. Nach vier Stunden überführt man weitere 5.79 ml (9.26 mmol) *n*-BuLi (1.6 M) in das Reaktionsgemisch und läßt 16 Stunden bei Raumtemperatur rühren. Zur Aufarbeitung wird die resultierende klare hellbraune Lösung auf 0°C abgekühlt und mit 2.2 eq. BH₃-THF Komplex (1M, 9.26 ml) versetzt. Nach 2 Stunden entfernt man das Lösungsmittel nimmt den Rückstand mit 20 ml Wasser auf und extrahiert das Produkt mit Methylenchlorid (3x 30 ml). Nach dem Einengen erhält man das gewünschte Produkt durch Reinigung mittels Säulenchromatographie (*n*-Hexan:AcOEt = 4:1, R_{f}=0.25). in einer Ausbeute von 480 mg (16 %). ¹H-NMR (CDCl₃): 7.38-7.20 (20 H, m, arom. H), 4.54-4.39 (8H, m, 4xCH₂Ph), 3.92-3.84 (4H, m, CH-O). 2.55 (2H, m, CH-P), 2.24 (2H, m, CH-P), 2.00-1.64 (4H, m, (CH₂)₂), 1.26-1.10 (12H, m, CH₃), 0.90--0.05 (6H, breit, BH₃); ¹³C-NMR (CDCl₃): 137.8, 137.5, 128.5-127.4 (arom. C), 84.0+82.3 (C-3+C-4), 72.5+72.3 (CH₂Ph), 34.3 (m, C-2+C-5), 17.8 (CH₂)₂, 10.0 (CH₃), 8.1 (CH₃); ³¹P-NMR (CDCl₃): 39.1;

1,2-Bis((*2S,3S,4S,5S*)-3,4-dibenzyloxy-2, 5-dimethyl-phospholanyl)ethan (2): 290 mg (0.41 mM) der Verbindung 1 werden mit 3 äq. DABCO in 15 ml Toluol bei 40°C gerührt. Nach Anzeige des vollständigen Umsatzes im DC (*n*-Hexan:AcOEt= 4:1, R_{f}=0.3) erhält man nach der säulenchromatographischen Aufarbeitung in einer Ausbeute von 75 % (210 mg) die Verbindung 2. ¹H-NMR (CDCl₃): 7.40-7.10 (20 H, m, arom. H), 4.54-4.34 (8H, m, 4xCH₂Ph), 3.96-3.78 (4H, m, CH-O), 2.53 (2H, m, CH-P), 2.10 (2H, m, CH-P), 1.73-1.50 (4H, m, (CH₂)₂), 1.30-1.05 (12H, m, CH₃) ; ¹³C-NMR (CDCl₃): 138.7, 138.5, 128.3-127.3 (arom. C), 86.8+85.1 (C-3+C-4), 72.2+71.9 (CH₂Ph), 33.5 (m, C-2+C-5), 22.6 (CH₂)₂, 14.1 (CH₃), 9.4 (CH₃); ³¹P-NMR (CDCl₃): -1.5;

Rh[1,2-Bis((*2S*,*3S*,*4S*,*5S*)-3,4-dibenzyloxy-2,5-dimethyl-phospholanyl)ethan][COD]tetrafluoroborat (3): Zu einer Lösung von 180 mg (0.26 mmol) des Bisphospholans 2 in 3 ml THF gibt man ein Äquivalent (107 mg) [Rh(COD)₂]BF₄ und rührt eine Stunde bei Raumtemperatur. Anschließend versetzt man die Lösung mit 15 ml Diethylether wobei sich ein dunkelbraunes Öl abscheidet. Nach Abtrennen der überstehenden Lösung wäscht man den zähen Rückstand mit Diethylether (3x10 ml), wobei sich ein fester Niederschlag bildet. Man trocknet anschließend unter Vakuum. Dabei erhält man den gewünschten Komplex als orange-braunes Pulver in einer Ausbeute von 134 mg (52 %). ¹H-NMR (CHCl₃): 7.40-7.10 (20 H, m, arom. H), 5.27 (2H, m, CH(COD)), 5.14 (2H, m, CH(COD)), 4.58-4.40 (8H, m, 4xCH₂Ph), 4.10-3.93 (4H, m, H-2+H-5), Rest nicht auswertbar; ¹³C-NMR (CDCl₃): 137.6, 137.2, 128.7-127.5 (arom. C), 101.8 (m, COD), 95.5 (m, COD), 83.8+83.5 (C-3+C-4), 73.0+72.6 (4xCH₂Ph), 41.9+33.5 (m, C-2+C-5), 14.5 (CH₃), 13.4 (CH₃); ³¹P-NMR (CDCl₃): 72.7 (d, ²J_{Rh,P}= 150 Hz);

### Beispiel 2

### Typische Vorschrift zur asymmetrischen Hydrierung:

In einem Laborautoklaven der Fa. Roth werden unter Schutzgas 0.01 mmol des Katalysators in MeOH vorgelegt, mit 10 mmol Substrat versetzt und bei 1 bar Wasserstoffdruck und Raumtemperatur hydriert. Die Ergebnisse und Hydrierzeiten sind aus nachstehender Tabelle ersichtlich.

### Hydrierergebnisse

Bedingungen : 25°C, 1 bar H₂, 10 mmol Substrat, 0.01 mol% Rh-Katalysator (8) gemäß Beispiel 1, MeOH

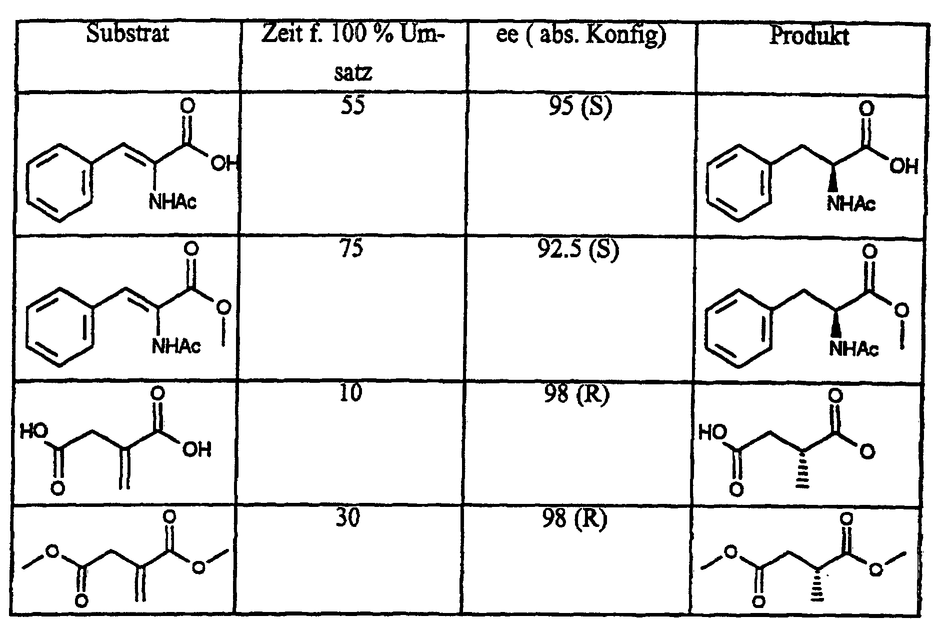

### Analytik:

Wurden die prochiralen Säuren AH bzw. ItH2 hydriert, so wurde eine kleine Menge (ca. 1 ml Lösung) mit Diazomethan bzw. Trimethylsilyldiazomethan verestert. Die nun vorhandenen Methylester aller Hydrierungsreaktionen wurden wie folgt analysiert: AH/AMe: (R/S)-2-Acetylamino-3-phenyl-propionsäuremethylester
- Säule:: 5 m XE 60 L-Valin tert. butylamid (0.2 mm Durchmesser) 165°C, Argon als TG (1ml/min), FID Retentionszeiten
R-Enantiomer: 4.3 min
S-Enantiomer: 4.6 min
- ItH2/ItMe2:: (R/S)-2-Methyl-bernsteinsaeuremethylester
- Säule:: 25 m Lipodex E (MACHEREY + NAGEL) 85°C, Argon als TG (1ml/min), FID Retentionszeiten
S-Enantiomer: 11.67 min
R-Enantiomer: 12.23 min

### Asymmetrische Hydrierung

Bedingungen: 1 bar H₂, 15 ml MeOH, 25°C, 1 mmol Substrat, 0.01 mmol Katalysator (3);

| | | |
|---|---|---|
| AH | 93.1 %*ee* (S)-Produkt | t_{1/2}= 31 min |
| AMe | 97.5 %*ee* (S)-Produkt | t_{1/2}= 26 min |

## Patentansprüche

1. Phospholane und Diphospholane der allgemeinen Formel I 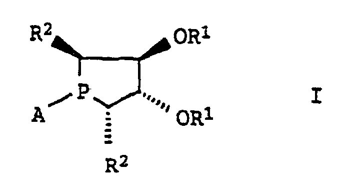 wobei:
R¹ und R² unabhängig voneinander C₁-C₆ -Alkyl, Aryl, Alkylaryl,
R¹ außerdem Wasserstoff,
A entweder R¹ oder 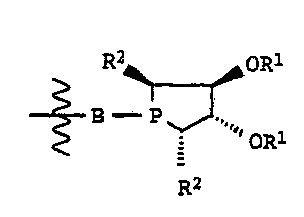
B ein Brückenglied mit 1 - 5 C-Atomen zwischen den beiden P-Atomen bedeuten.

2. Phospholane nach Anspruch 1, **dadurch gekennzeichnet, daß** die Substituenten die folgende Bedeutung besitzen:
R¹ Benzyl, Methyl, tert.Butyl oder zusammen mit dem anderen Substituent R¹ Isopropyliden oder Benzyliden.

3. Diphospholane nach Anspruch 1, **dadurch gekennzeichnet, daß** die Substituenten die folgende Bedeutung besitzen: 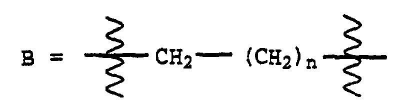 mit n = 0, 1, 2, 3, 4 oder 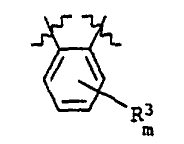 mit
m = 0, 1, 2, 3
R³ = Alkyl oder annelliertes Aryl

4. Diphospholane nach Anspruch 3, **dadurch gekennzeichnet, daß** die Substituenten die folgende Bedeutung besitzen:
m=0, n=1.

5. Metallkomplexe aus Phospholanen gemäß einem der Ansprüche 1 - 4 und einem Zentralatom ausgewählt aus der Gruppe Rh, Ru, Ir, Pd, Pt, Ni.

6. Metallkomplexe nach Anspruch 5, **dadurch gekennzeichnet daß** als Zentralatom Rh oder Ru ausgewählt ist.

7. Metallkomplexe nach Anspruch 6, **dadurch gekennzeichnet, daß** als Phospholan oder Diphospholan gemäß Anspruch 2 - 4 ausgewählt wird.

8. Verwendung von Metallkomplexen gemäß Anspruch 5 - 7 zur asymmetrischen Hydrierung, Hydroformylierung, Hydrocyanierung, allylischen Substitution, Isomerisierung von Allylaminen zu Enaminen.

9. Verfahren zur asymmetrischen Hydrierung von Verbindungen indem man die zu hydrierenden Ausgangsverbindungen mit Wasserstoff in Gegenwart der Metallkomplexe gemäß Anspruch 5 - 7 umsetzt.

10. Verfahren nach Anspruch 9, **dadurch gekennzeichnet, daß** die Hydrierung bei einem Wasserstoffdruck von 1 bis 2 bar ausgeführt wird.

## Claims

1. A phospholane or diphospholane of the formula I 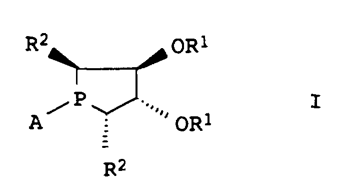 where
R¹ and R² are, independently of one another, C₁-C₆-alkyl, aryl, alkylaryl,
R¹ is also hydrogen,
A is either R¹ or 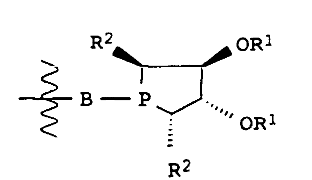
B is a linker having 1 - 5 carbon atoms between the two phosphorus atoms.

2. A phospholane as claimed in claim 1, wherein the substituents have the following meaning:
R¹ benzyl, methyl, tert-butyl, or together with the other substituent R¹, isopropylidene or benzylidene.

3. A diphospholane as claimed in claim 1, wherein the substituents have the following meaning: 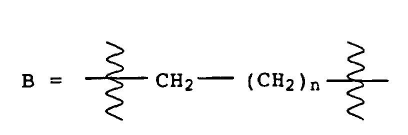 with n = 0, 1, 2, 3, 4 or 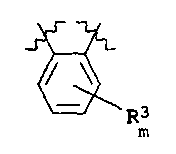 with m = 0, 1, 2, 3
R³ = alkyl or fused aryl

4. A diphospholane as claimed in claim 3, wherein the substituents have the following meaning:
m=0, n=1

5. A metal complex of a phospholane as claimed in any of claims 1 - 4 and a central atom selected from the group of Rh, Ru, Ir, Pd, Pt, Ni.

6. A metal complex as claimed in claim 5, wherein Rh or Ru is selected as central atom.

7. A metal complex as claimed in claim 6, wherein the phospholane or diphospholane is selected from those claimed in claims 2 - 4.

8. The use of a metal complex as claimed in claim 5 - 7 for asymmetric hydrogenation, hydroformylation, hydrocyanation, allylic substitution, isomerization of allylamines to enamines.

9. A process for the asymmetric hydrogenation of compounds by reacting the starting compounds which are to be hydrogenated with hydrogen in the presence of a metal complex as claimed in claim 5 - 7.

10. A process as claimed in claim 9, wherein the hydrogenation is carried out under a hydrogen pressure of from 1 to 2 bar.

## Revendications

1. Phospholanes et diphospholanes de formule générale I 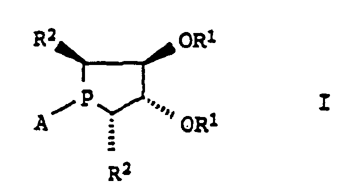 dans laquelle
R¹ et R² représentent chacun, indépendamment l'un de l'autre, un groupe alkyle en C1-C6, aryle, alkylaryle,
R¹ pouvant en outre représenter l'hydrogène,
A a les mêmes significations que R¹ ou représente 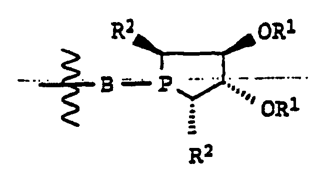 dans lequel
B représente un pont en C1-C5 entre les deux atomes de phosphore.

2. Phospholanes selon la revendication 1, **caractérisés par le fait que** les symboles ont les significations suivantes :
R¹ représente un groupe benzyle, méthyle ou tert-butyle ou forme avec l'autre substituant R¹ un groupe isopropylidène ou benzylidène.

3. Diphospholanes selon la revendication 1, **caractérisés par le fait que** les symboles ont les significations suivantes : 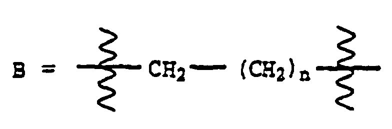 avec n = 0, 1, 2, 3, 4 ou 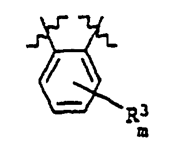 avec
m = 0, 1, 2, 3
R³ = alkyle ou aryle condensé

4. Diphospholanes selon la revendication 3, **caractérisés par le fait que** les symboles ont les significations suivantes :
m = 0, n = 1,

5. Complexes métalliques de phospholanes selon l'une des revendications 1 à 4 ayant un atome central choisi dans le groupe formé par Rh, Ru, Ir, Pd, Pt, Ni.

6. Complexes métalliques selon la revendication 5, **caractérisés par le fait que** l'atome central consiste en Rh ou Ru.

7. Complexes métalliques selon la revendication 6, **caractérisés par le fait que** le phospholane ou diphospholane est choisi parmi les phospholanes ou diphospholanes selon les revendications 2 à 4.

8. Utilisation de complexes métalliques selon les revendications 5 à 7 pour l'hydrogénation asymétrique, l'hydroformylation, l'hydrocyanation, la substitution allylique, l'isomérisation des allylamines en énamines.

9. Procédé pour l'hydrogénation asymétrique de composés dans lequel on fait réagir les composés de départ à hydrogéner avec l'hydrogène en présence de complexes métalliques selon les revendications 5 à 7.

10. Procédé selon la revendication 9, **caractérisé par le fait que** l'hydrogénation est réalisée sous une pression d'hydrogène de 1 à 2 bar.
